# EUROPEAN PATENT SPECIFICATION

(11) **EP 0 898 939 B1**
(45) Date of publication and mention of the grant of the patent: **14.01.2004**
(21) Application number: 98115542.7
(22) Date of filing: 18.08.1998
(51) Int. Cl.: A61B 17/06

(54) **Suture Needle**
Chirurgische Nähnadel
Aiguille de suture chirurgicale

(30) Priority: 29.08.1997 JP 23363297
(43) Date of publication of application: 03.03.1999
(73) Proprietor: Mani, Inc., Shioya-gun, Tochigi-ken 329-1234 (JP)
(72) Inventor: Matsutani, Kanji, c/o MANI INC., Shioya-gun, Tochigi-ken, 329-1234 (JP); Matsumoto, Takayuki, c/o MANI INC., Shioya-gun, Tochigi-ken, 329-1234 (JP)
(74) Representative: Grünecker, Kinkeldey, Stockmair & Schwanhäusser Anwaltssozietät

(56) References cited:
- WO-A-95/23876
- US-A- 5 464 422
- PATENT ABSTRACTS OF JAPAN vol. 015, no. 248 (C-0843), 25 June 1991 -& JP 03 080869 A (MATSUTANI SEISAKUSHO CO LTD), 5 April 1991

## Description

### FIELD OF THE INVENTION

The present invention relates to a suture needle for medical use.

### DESCRIPTION OF THE RELATED ART

Many kinds of medical suture needles have been used in accordance with their usage, and dull needles, square needles, and round needles have representatively been utilized.

A dull needle is used for suturing lever and the like, and a tip of the needle is dull. A square needle is generally used for suturing hard tissue such as skin and muscle, and has a sharp tip and a tapered portion with a shape of a polygonal pyramid, and each prescribed edge on the tapered portion is used as a cutting edge to cut and open a tissue.

A round needle is provided with a body with an appropriate cross-section and a conically tapered portion on the side of a tip thereof. Although the shape of the cross-sections of the conically tapered portion and the body are usually round, in place of the round cross-section, an oval which is sandwiched between two substantially parallel planes, a cross-section enclosed by four planes, and a cross-section which is sandwiched between two substantially parallel planes and of which central portion is constricted may be adopted. However, the tapered portion of a round needle is not adapted to be used as a cutting edge, which is obtained by sharpening an edge like a square needle.

After drilling a tissue with a tip, a round needle enlarges the drilled hole with the tapered portion. Unlike a square needle, a round needle is not provided with a cutting edge at the tapered portion so that the needle does not cut a tissue unnecessarily. Therefore, a tissue at the hole closely contacts the surface of a thread, which prevents body fluid and the like from being spilt from the sutured portion. With this characteristics, a round needle is mainly used for suturing blood vessels and soft tissue.

The above-mentioned round needle is manufactured in the following manner.

At first, a linear material with a prescribed diameter is cut in a predetermined length. Then, a portion engaging a suture thread is formed at an end of the linear material. As the engaging portion, for instance, a spring eye , or a blind hole which is drilled in an axial direction of the linear material is adopted. Next, a tip and an intermediate portion of the material are ground with a whetstone or the like to form a sharp tip and a tapered portion. Then, rough buff polishing and another polishing with fine whetstone or the like, grinding stripes are removed through buff polishing, electrolytic polishing or the like to make mirror-finished surface, and the liner material is bent to form a prescribed shape, and is heat-treated and surface-treated to obtain the round needle.

In this connection, a demand for the sharpness of a needle, that is, the reduction of impalement resistance has been considerably becoming strong. However it is difficult to increase the cutting quality of a round needle without cutting edge In case of a square needle such as a triangular suture needle, as described above, edges at the pyramid-shaped tapered portion function as cutting edges to open a tissue while the needle proceeds in the tissue, so that it is relatively easy to reduce impalement resistance. But, in such a case. the square needle opens tissue with the edges thereof. Therefore, cut section becomes large to cause sealing property after an impalement of the needle to decrease. resulting in spilt body fluid. Therefore, the square needle is not suitable for the suture of blood vessels and the like.

To solve the above-mentioned problems. in a prior art, the surface of the needle is mirror-finished to reduce the impalement resistance. That is. as the surface treatment in the above-mentioned manufacturing process. finishing process through buff polishing, electrolytic polishing, chemical polishing or the like was carried out to form a mirror surface.

In the finishing process through buff polishing, cotton cloth, felt, or the like with fine abrasive grain is rotated and is pressed to a ground material to cause the abrasive grain to polish and finish the material, which allows a tip and a body of a needle to be mirror-finished.

In the finishing process through electrolytic polishing, electricity is forced to be applied to a needle to melt the surface of the needle through electrolysis.

In the finishing process through chemical grinding, unlike the finishing process through electrolytic grinding, electricity is not forced to be applied, but acid causes the surface of a needle to be melt. In this case also, polish and finished surface becomes mirror surface.

With the above-mentioned finishing processes, although finished surfaces are smooth mirror surface with naked eyes. they are not mirror surfaces microscopically. For instance. with the buff finishing, many rough stripes caused by abrasive grain are observed.

Further, with the electrolytic grinding, microscopically, it is confirmed that gases generated at the electrolysis adhere to the surface to form rough surface with shallow craters.

With the chemical grinding, besides the rough surface with craters caused by the gases like the electrolytic polishing, further shallow roughness is formed on the surface of a needle by easily ground crystal grains and hardly ground crystal grains of the material of the needle.

As described above, there are all kinds of mirror-finishing, and whithout complete mirror surface or not should be discussed, in other words, the degree of the mirror finishing varies with material to be ground. For example, mirror-finishing for silicon wafer and that for plate member for constructing buildings are different from each other. In the detailed description of the invention, mirror surface is defined to be such finished surface of a needle as finished by generally used buff polishing, electrolytic polishing, or chemical polishing.

With a more microscopically smooth mirror surface of a needle, however, the impalement resistance of the needle could not be reduced. In other words, there was no difference between mirror surface through buff polishing, electrolytic polishing, chemical polishing, and more smooth mirror surface.

In another method of decreasing the impalement resistance, a tapered portion was coated with silicon. But, in this case, the silicon was peeled off after several impalements, the effect of the silicon was remarkable decreased.

To solve the above-mentioned problem, in Japanese Patent Publication No. Heisei 5-18576, it is proposed that channels with craters are formed on a tapered portion through chemical grinding, and silicon is applied onto the portion to manufacture a needle. With such construction, silicon is sustained in the crater-like concave portions, and even after the needle is repeatedly used, the increase in the impalement resistance may be prevented.

However, in a suture needle in which silicon is sustained in the crater-like concave portions formed by the chemical polishing, as described above, the concave portions are remarkably shallow as generally called as mirror surface, the silicon is not so effective.

Further, in Japanese Patent Publication No. Heisei 5-60746, in order to reduce the impalement resistance, a tapered portion near the tip of a needle is formed to be long to change taper ratio (that is, the portion is formed to be thin and sharp).

However, when the taper ratio is changed, as explained below, it is effective to a thin tissue but is not effective to a thick tissue.

Generally, it is known that there are two peaks of impalement resistance of a round needle. The first peak is observed when the tip of the needle enters a tissue, and the second peak is observed when the end of the tapered portion (the thickest portion) enters the tissue. In other words, the moment the tip of the needle just enters a tissue, the first peak is observed, and after that, the resistance is lowered once. Then, the resistance value gradually increases as the tapered portion becomes thick, and when the end of the tapered portion, that is, when the thickest portion passes enters the tissue, the second peak is observed.

A sharper tip of a needle causes the first peak to be decreased. In other words, if a tissue is so thin that the tip of the tapered portion penetrates the tissue, the sharper tip is effective. But, when the tissue is thick in comparison to the long tapered portion, not only the long gentle tapered portion but also a short steep tapered portion contacts the tissue from the tip to the end of the needle, so that work load of the both tapered portions are the same (impalement resistance here is calculated by the following formula: impalement resistance = the coefficient of friction x pressure x distance that the tapered portion contacts the tissue). The change in the taper ratio allows the "pressure" to be decreased, but "the distance that the tapered portion contacts the tissue" becomes long.), therefore, the sharper tip is not effective. This is applied not only to a round needle but to a needle with cutting edge only at the tip, so-called a cutting tapered needle. That is, the above thing is applied to all needles without cutting edge at the tapered portion where the cross-section of the needle becomes large.

WO 9523876 describes a method of treatment of a shape memory alloy which involves shot peening of the alloy sample to modify the structure of the surface layer of the sample without substantially affecting bulk characteristics of the material. US 5,464,422 describes a suture needle having, in sequence, a shaft portion, a tip portion, a conical point portion and a point.

### SUMMARY OF THE INVENTION

The present invention has been made in consideration of the above-mentioned fact and, it is therefore an object of the present invention to provide a suture needle in which impalement resistance is further decreased in comparison to a conventional one.

To achieve the above object, there is provided the suture needle according to the present invention as defined in claim 1 comprising a base end portion, at an end of the suture needle, engaging a suture thread, a tip, at another end of the suture needle, impaling a tissue, and a tapered portion reaching to the tip (see e.g. JP-A-03 080 869), characterized in that the tapered portion is provided with a roughness of which stripe extends in a substantially axial direction thereof. This stripe roughness is rougher and longer than the mirror surface of the suture needle described above. This stripe roughness is formed by grinding with whetstone or the like, and rougher surface compared with this stripe roughness is excluded.

It is possible to mirror-finish only the tip portion of the tapered portion, or form cutting edge to the portion, or apply silicon treatment to the tapered portion.

Further, it is also possible that the tapered portion is not provided with a cutting edge; the stripe roughness is formed by grinding; the stripe roughness is more than twice as rough as that of a mirror surface; the stripe roughness is polished to a degree that the stripe does not disappear; a portion of the tapered portion near the tip of the suture needle has mirror surface; a portion of the tapered portion near the tip of the suture needle has a cutting edge; silicon treatment is applied to the tapered portion; length, in an axial direction of the suture needle, of the mirror surface at the portion of the tapered portion near the tip of the suture needle is 5% to 20% of length, in an axial direction, of the tapered portion; and length, in an axial direction of the suture needle, of the cutting edge at the portion of the tapered portion near the tip of the suture needle is 5% to 20% of length, in an axial direction, of the tapered portion.

### BRIEF DESCRIPTION OF THE DRAWING

The present invention will be more apparent from the ensuring description with reference to the accompanying drawings, wherein:
Figure 1 is a perspective view showing overall suture needle according to the present invention;
Figure 2 is an enlarged view of the tapered portion and the base end portion, which are arranged in a row, of the suture needle shown in Fig. 1;
Figure 3 is a cross-sectional view taken along the line III-III of Fig. 2, but only upper half is illustrated;
Figure 4 shows the tip of the suture needle according to the second embodiment of the present invention;
Figure 5a and Figure 5b show the tip of the suture needle according to the third embodiment of the present invention;
Figure 6 is a drawing for explaining a method of measuring impalement resistance of a suture needle;
Figures 7 and 8 are graphs showing impalement resistance of the suture needles according to the present invention and conventional suture needles.

### DETAILED DESCRIPTION OF THE INVENTION

Next, embodiments of the suture needle according to the present invention will be explained with reference to drawings.

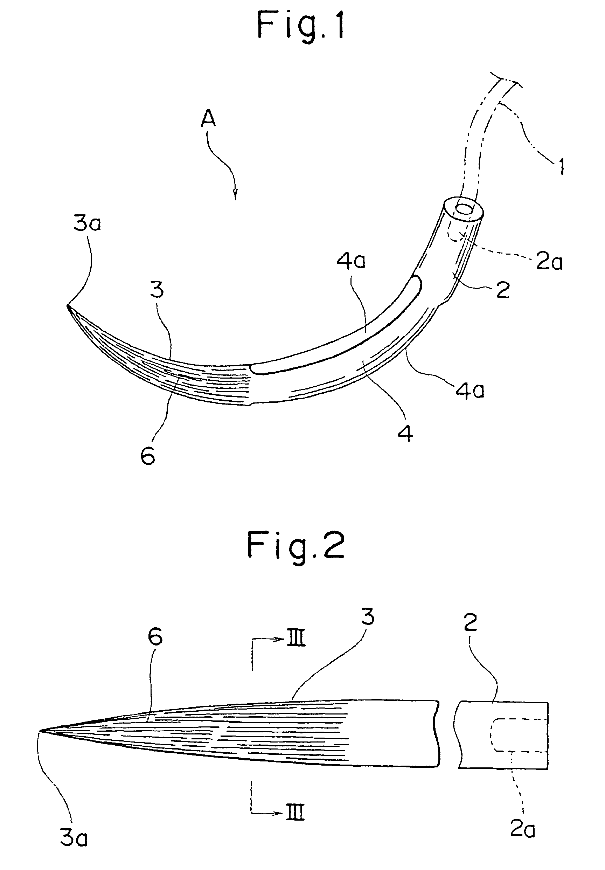

A suture needle A shown in Fig. 1 is a round needle, and a base end portion 2 engaging a suture thread 1 is provided at an end of the needle, and a blind hole 2a is drilled at the base end portion 2 in an axial direction of the needle. where the suture thread 1 is to be inserted and fixed.

At another end of the needle, a tapered portion 3, of which diameter becomes larger as apart from a tip 3a as a base point, is provided. "Tapered portion" used here is. as described above, defined to be general shape of which diameter or cross-section increases as apart from the tip of a needle. In this case, the change of the cross-section may be linear, as a matter of course, or curvilinear.

A portion between the base end portion 2 and tapered portion 3 is a body 4 of which diameter is substantially constant. In case of a round needle, the body 4 has a round cross-section, but ordinarily, two planes 4a, 4a which are in parallel with each other are formed through press so that the needle is easily sustained by a needle-carrier at suturing work. Further, it is possible to provide four planes to form a square, which causes the needle to be more easily sustained and the strength of the needle to be increased.

Although the above is also applied to a conventional round needle. a round needle according to the present invention is characterized in that stripe rough surface 6. of which stripes direct in a substantially axial direction of the needle through grinding, is provided on the tapered portion 3.

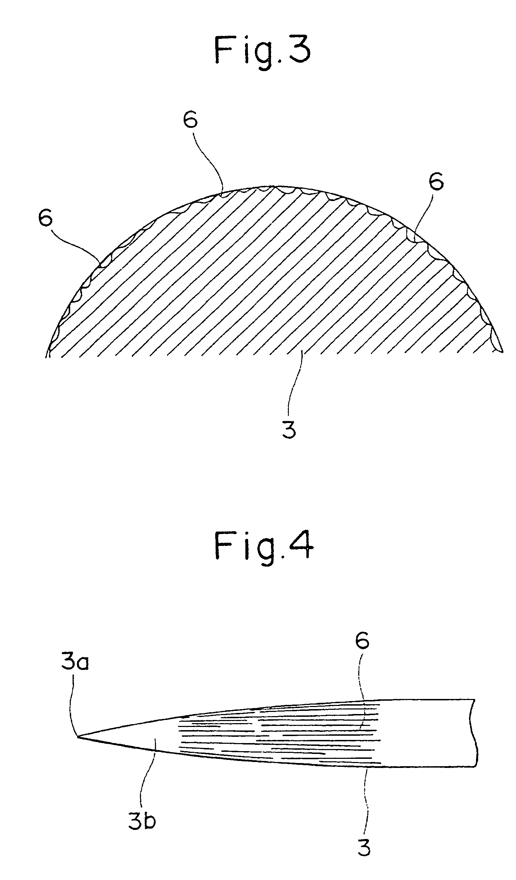

Figure 3 is an enlarged view of the tapered portion 3 of the suture needle shown in Fig. 2. As illustrated, the tapered portion 3 is provided with the stripe rough surface 6. This stripe rough surface is preferably formed by a whetstone regulated in ISO #30 to #320, a grinding belt, or the like. Further, the roughness of the stripe rough surface is preferably more than twice as rough as that of a mirror surface.

In the suture needle according to the above embodiment, when a tissue is pierced at a predetermined portion by the tip of the needle, a fine clearance is formed between the stripe rough surface 6 and the tissue, so that the area where the stripe rough surface 6 and the tissue contact with each other decreases, allowing the tissue to be impaled by the suture needle with small force. The clearance is very fine, and it is formed only when the tapered portion proceeds, which securely prevents body fluid from being spilt after the proceeding of the needle, unlike the conventional one. Further the size of the stripe roughness is larger than those formed by chemical polishing. With the crater-like roughness formed by chemical grinding, such fine clearances as described above are not generated, therefore, it is hardly expected to reduce the impalement resistance.

In this connection, the width or depth of the stripe roughness may freely be adjusted by selecting the number of abrasive grain for grinding and machining conditions. Further, when the stripe rough surface 6 is too rough or feel rough after grinding only, it is preferable to slightly carry out buff finishing, electrolytic polishing, chemical polishing, or the like after the grinding to smooth the stripe roughness.

Figure 4 shows a suture needle according to the second embodiment of the present invention. In the above-mentioned embodiment, burr generated at machining with abrasive grain causes the tip of a needle not to be formed sharp. Then, only a tip portion 3b is formed to be mirror surface like the conventional one. Finishing method is selected from the buff finishing, electrolytic polishing, and chemical polishing. Further, the length l of the tip portion to be finished is preferably substantially 5% to 20% of the length L of the tapered portion 3.

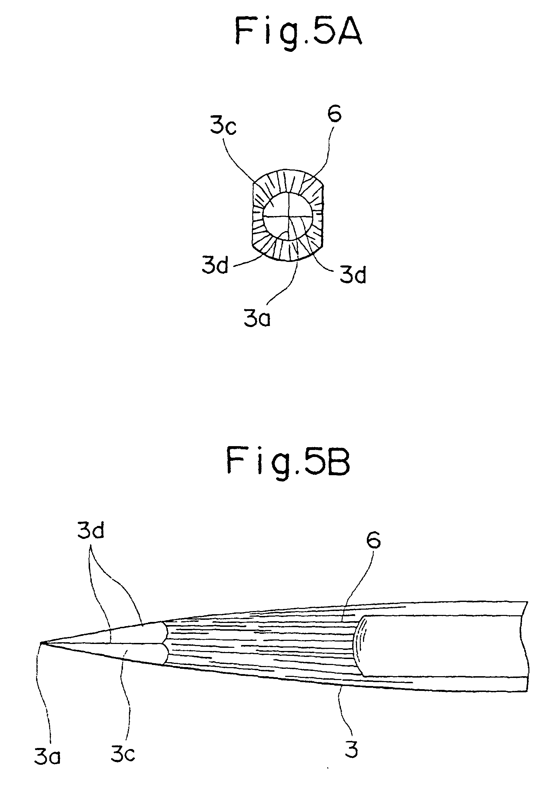

Figures 5(a) and 5(b) show a suture needle according to the third embodiment of the present invention. In this embodiment, only a tip portion 3c which is mirror-finished as shown in Fig. 4 is formed to be a pyramid. Each edge 3d of the pyramid becomes a cutting edge to open a tissue and reduce the impalement resistance. However, this cutting edge is formed only on the tip portion 3c, and after the tip portion 3c enters in the tissue, the stripe rough surface 6 impales the tissue. Like this, the pyramid is formed only on a limited tip portion to eliminate a harmful influence that the tissue is cut too widely like the conventional square needle, and moreover, the initial impalement resistance can be decreased.

In all the embodiments, conventionally used silicon treatment is preferably applied. With the silicon treatment, silicon enters the stripe roughness, so that the silicon is adequately maintained, which prevents the increase of the impalement resistance after repeated use of the suture needle.

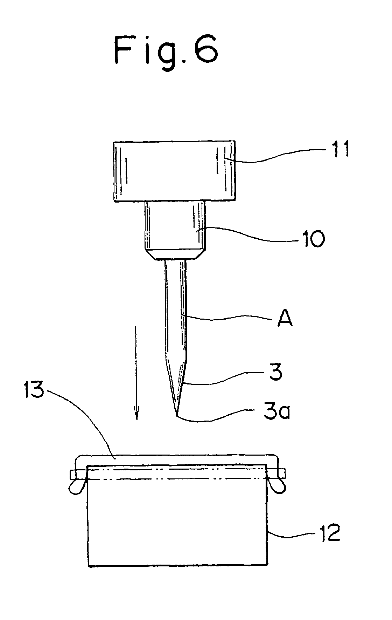

Figure 6 is a drawing for explaining a method of measuring the impalement resistance of the suture needles according to the present invention. The suture needle A is maintained in a linear shape before bending, and is cramped by a vertically movable chuck 10. The chuck 10 is provided with a load cell 11. An upwardly opened box 12 is located below the suture needle A, and the opening of the upwardly opened box 12 is covered by an impalement material 13 (polyurethane with a thickness of 0.45 mm or a film-shaped "porvair" with a thickness of 1.10 mm made by Dow Corning Co.).

The chuck 10 is lowered by a driver not shown, and the tip 3a of the suture needle A impales the impalement material 13. At this moment, the impalement resistance is measured by the load cell 11.

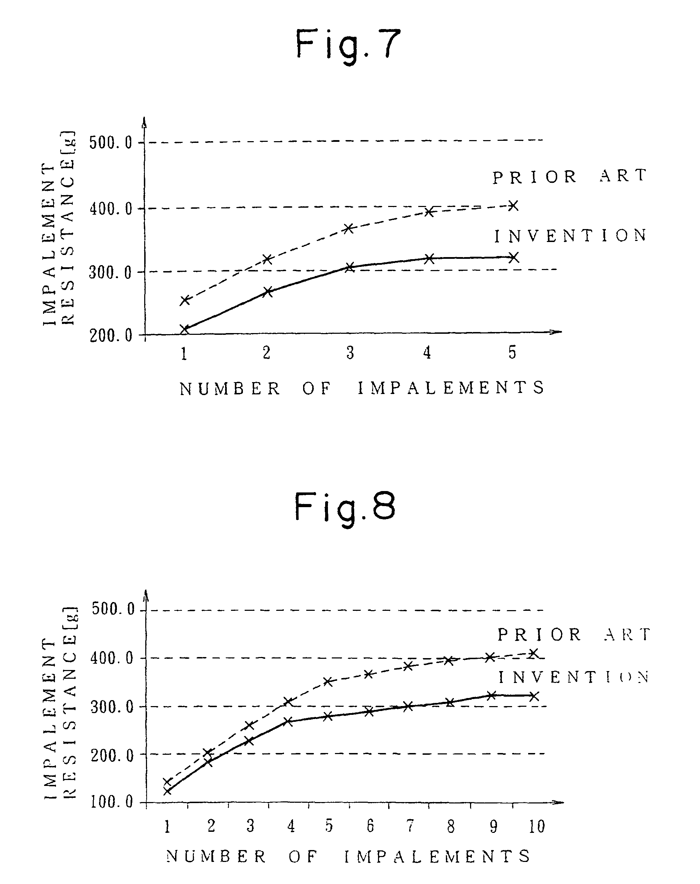

Figures 7 and 8 are graphs indicating the impalement resistance measured by the instrument illustrated in Fig. 6 when conventional suture needles and the suture needles according to the present invention are used. Solid lines show the impalement resistance when suture needles according to the present invention are used, and dotted lines show those of conventional suture needles. The suture needles according to the present invention have a stripe roughness (ground by the whetstone regulated in ISO #50) at a tapered portion, and a top is mirror-finished, and after that, chemical polishing is applied to overall suture needle. The conventional suture needle is mirror-finished at an ordinary tapered portion thereof through buff machining, and after that, chemical polishing is applied to overall suture needle. The suture needles shown in Fig. 7 are not subject to silicon treatment, but the suture needles in Fig. 8 are treated by silicon ("MDX-4159" made by Dow Corning Co.) after the same process to the suture needles in Fig. 7. The depth of the stripe roughness of the suture needle according to the present invention is approximately 40µm. The surface roughness of the mirror surface of the suture needle is approximately 8µm.

The suture needles without silicon treatment impaled the impalement material 13 five times, and the impalement resistance at each impalement was measured with the load cell. The suture needles with silicon impaled ten times, and the impalement resistance at each impalement was measured.

In the both cases in Figs. 7 and 8, it is clearly recognized that the suture needles according to the present invention are superior to conventional suture needles. Especially, as shown in Fig. 8. there is not much difference between the both suture needles with silicon until four impalements, but, after that, the difference becomes larger as the number of impalements increases. This is because repeated impalements causes silicon applied to the conventional suture needle to gradually be peeled off, but. in the present invention, silicon is sustained on the stripe roughness and the effect of the silicon treatment continues.

The above explanation was made for a round suture needle, but it is possible to form the stripe roughness through grinding according to the present invention to tapered portions of a square needle and a dull needle and the effect thereof may be obtained. And the same effect can be obtained even if the engaging portion with a suture thread was a blind hole or a spring eye: the shape of cross-section of a body was oval or a cross-section which is sandwiched between two substantially parallel planes and a central portion is constricted; and the needle was bent or straight. But, the above effect is remarkable when the suture needle is not provided with cutting edge at a tapered portion thereof.

As described above, with the present invention, a suture needle comprising a base end portion, at an end of the suture needle, engaging a suture thread, a tip, at another end, impaling a tissue, a tapered portion reaching to the tip, is characterized in that the tapered portion is provided with stripe roughness through grinding, so that fine clearances are formed between the stripe roughness and the tissue, resulting in reduced impalement resistance.

## Claims

1. A suture needle (A) comprising:
a base end portion (2), at an end of said suture needle (A), engaging a suture thread (1);
a tip (3a), at another end of said suture needle (A), for impaling a tissue; and
a tapered portion (3) reaching to said tip (3a),
**characterized in that**
said tapered portion (3) having a rough surface comprising stripes (6) extending in a substantially axial direction of said tapered portion and formed by grinding,
wherein said rough surface comprising stripes (6) is more than twice as rough as that of a mirror-finished surface obtained by buff finishing, electrolytic polishing, or chemical polishing.

2. The suture needle (A) as claimed in claim 1, wherein said tapered portion (3) includes no-cutting-edge tapered portion.

3. The suture needle (A) as claimed in claim 1, wherein said rough surface comprising stripes (6) is polished to a degree that said stripe does not disappear.

4. The suture needle (A) as claimed in claim 1, wherein a tip portion (3b) of said tapered portion (3) has mirror-finished surface.

5. The suture needle (A) as claimed in claim 1, wherein a tip portion (3c) of said tapered portion (3) has a cutting edge (3d).

6. The suture needle (A) as claimed in claim 1, wherein silicon treatment is applied to said tapered portion (3).

7. The suture needle (A) as claimed in claim 4, wherein a length, in an axial direction of the suture needle (A), of said mirror-finished surface at said portion (3b) of said tapered portion (3) near the tip (3a) is 5% to 20% of length, in an axial direction, of said tapered portion (3).

8. The suture needle (A) as claimed in claim 5, wherein a length, in an axial direction of the suture needle (3), of said cutting edge (3d) at said portion (3c) of said tapered portion (3) near the tip (3a) is 5% to 20% of length, in an axial direction, of said tapered portion (3).

## Patentansprüche

1. Nähnadel (A), umfassend:
einen Basisendabschnitt (2) an einem Ende der Nähnadel (A), um einen Nähfaden (1) aufzunehmen;
eine Spitze (3a) an einem anderen Ende der Nähnadel (A), um ein Gewebe durchzustechen; und
einen konisch zulaufenden Bereich (3), der zu der Spitze (3a) reicht,
**dadurch gekennzeichnet, dass**
der konisch zulaufenden Bereich (3) eine raue Oberfläche mit Streifen umfasst, die sich in einer im wesentlichen axialen Richtung des konisch zulaufenden Bereichs erstrecken, und die durch Schleifen erzeugt werden,
worin die Streifen umfassende raue Oberfläche (6) mehr als zweimal so rau ist wie die einer spiegelnd geglätteten Oberfläche, die man mit Glanzschleifenglättung, elektrolytischem Polieren oder chemischen Polieren erhält.

2. Nähnadel (A) nach Anspruch 1, worin der konisch zulaufenden Bereich (3) einen konisch zulaufenden Bereich mit nicht schneidenden Kanten einschließt.

3. Nähnadel (A) nach Anspruch 1, worin die Streifen umfassende raue Oberfläche (6) in einem Ausmaß poliert wird, dass die Streifen nicht erscheinen.

4. Nähnadel (A) nach Anspruch 1, worin ein Spitzenbereich (3b) des konisch zulaufenden Bereichs (3) eine spiegelnd geglättete Oberfläche aufweist.

5. Nähnadel (A) nach Anspruch 1, worin ein Spitzenbereich (3c) des konisch zulaufenden Bereichs (3) eine Schneidkante (3d) aufweist.

6. Nähnadel (A) nach Anspruch 1, worin der konisch zulaufenden Bereich (3) mit Silikon behandelt ist.

7. Nähnadel (A) nach Anspruch 4, worin eine Länge der spiegelnd geglätteten Oberfläche in axialer Richtung der Nähnadel (A) an dem Bereich (3b) des konisch zulaufenden Bereichs (3) nahe der Spitze (3a) 5% bis 20% der Länge des konisch zulaufenden Bereichs (3) in axialer Richtung ist.

8. Nähnadel (A) nach Anspruch 5, worin eine Länge der Schneidkante (3d) in axialer Richtung der Nähnadel (A) an dem Bereich (3b) des konisch zulaufenden Bereichs (3) nahe der Spitze (3a) 5% bis 20% der Länge des konisch zulaufenden Bereichs (3) in axialer Richtung ist.

## Revendications

1. Aiguille à suture (A) comprenant :
une partie terminale de base (2) à une extrémité de ladite aiguille à suture (A), engageant un fil de suture (1) ;
une pointe (3a) à l'autre extrémité de ladite aiguille à suture (A) pour pénétrer dans un tissu ;et
une partie conique (3) en allant vers ladite pointe (3a),
**caractérisée en ce que**
ladite partie conique (3) ayant une surface rugueuse comportant des bandes (6) s'étendant en une direction sensiblement axiale par rapport à ladite partie conique et formées par meulage,
dans laquelle ladite surface rugueuse comportant des bandes (6) est plus de deux fois plus rugueuse que celle d'une surface polie en miroir obtenue par polissage au feutre, polissage électrolytique ou polissage chimique.

2. Aiguille à suture (A) selon la revendication 1 dans laquelle ladite partie conique (3) inclut une partie conique à bord non tranchant.

3. Aiguille à suture (A) selon la revendication 1, dans laquelle ladite surface rugueuse comportant des bandes (6) est polie à un degré tel que lesdites bandes ne disparaissent pas.

4. Aiguille à suture (A) selon la revendication 1, dans laquelle une partie de pointe (3b) de ladite partie conique (3) a une surface polie en miroir.

5. Aiguille à suture (A) selon la revendication 1, dans laquelle une partie de pointe (3c) de ladite partie conique (3) a un bord tranchant (3d).

6. Aiguille à suture (A) selon la revendication 1, dans laquelle un traitement à la silicone est appliqué à ladite partie conique (3).

7. Aiguille à suture (A) selon la revendication 4 dans laquelle une longueur, dans une direction axiale de l'aiguille à suture (A), de ladite surface polie en miroir au niveau de ladite partie (3b) de ladite partie conique (3) à proximité de la pointe (3a) a une longueur, en direction axiale, correspondant à 5 à 20 % de ladite partie conique (3).

8. Aiguille à suture (A) selon la revendication 5, dans laquelle une longueur, dans une direction axiale de l'aiguille à suture (3), dudit bord tranchant (3d) au niveau de la partie (3c) de ladite partie conique (3) à proximité de la pointe (3a) a une longueur, en direction axiale, correspondant à 5 à 20 % de ladite partie conique (3).
